# EUROPEAN PATENT SPECIFICATION

(11) **EP 2 512 240 B1**
(45) Date of publication and mention of the grant of the patent: **11.04.2018**
(21) Application number: 10838320.9
(22) Date of filing: 17.12.2010
(51) Int. Cl.: A61K 31/192, A61K 31/195, A61K 9/06, A61P 29/00

(54) **METHODS AND COMPOSITIONS FOR TREATING AND PREVENTING TRIGEMINAL AUTONOMIC CEPHALGIAS, MIGRAINE, AND VASCULAR CONDITIONS**
VERFAHREN UND ZUSAMMENSETZUNGEN ZUR BEHANDLUNG UND PRÄVENTION VON TRIGEMINOAUTONOMEN ZEPHALGIEN, MIGRÄNE UND GEFÄSSERKRANKUNGEN
MÉTHODES ET COMPOSITIONS DE TRAITEMENT ET DE PRÉVENTION DES CÉPHALÉES TRIGÉMINO-AUTONOMIQUES, DES MIGRAINES ET DES PATHOLOGIES VASCULAIRES

(30) Priority: 18.12.2009 US 287953 P
(43) Date of publication of application: 24.10.2012
(73) Proprietor: Achelios Therapeutics, Inc., Chapel Hill, North Carolina 27517 (US)
(72) Inventor: LEIGHTON, Harry, J., Rockport ME 04856 (US); BUDERER, Matthew, J., Oak Harbor OH 43449 (US); FRANGAKIS, Crist, J., Chapel Hill NC 27517 (US)
(74) Representative: Bösl, Raphael Konrad
(86) International application number: PCT/US2010/061130
(87) International publication number: WO 2011/075688

(56) References cited:
- US-A1- 2002 099 060
- US-A1- 2002 143 047
- US-A1- 2003 119 892
- US-A1- 2004 138 239
- US-A1- 2006 178 354
- US-A1- 2007 093 420
- US-A1- 2008 279 895
- US-A1- 2009 123 528
- US-A1- 2009 214 466
- US-A1- 2009 258 884
- US-A1- 2009 281 080
- US-A1- 2009 312 435
- US-B1- 6 221 377
- US-B1- 6 387 407
- DATABASE MEDLINE [Online] US NATIONAL LIBRARY OF MEDICINE (NLM), BETHESDA, MD, US; August 1996 (1996-08), GÖBEL H ET AL: "[Effectiveness of Oleum menthae piperitae and paracetamol in therapy of headache of the tension type].", XP002722303, Database accession no. NLM8805113 & GÖBEL H ET AL: "[Effectiveness of Oleum menthae piperitae and paracetamol in therapy of headache of the tension type].", DER NERVENARZT AUG 1996, vol. 67, no. 8, August 1996 (1996-08), pages 672-681, ISSN: 0028-2804
- HEIR G ET AL: "Use of topical medication in orofacial neuropathic pain: a retrospective study", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 105, no. 4, April 2008 (2008-04), pages 466-469, XP022585079, ISSN: 1079-2104, DOI: 10.1016/J.TRIPLEO.2007.09.030 [retrieved on 2008-03-06]
- BROWN ET AL: "Prescribing flexibility through prescription compounding", TECHNIQUES IN REGIONAL ANESTHESIA AND PAIN MANAGEMENT, W.B. SAUNDERS, AMSTERDAM, NL, vol. 12, no. 2, April 2008 (2008-04), pages 119-121, XP022593254, ISSN: 1084-208X, DOI: 10.1053/J.TRAP.2008.02.001 [retrieved on 2008-04-10]
- MARTELLETTI PAOLO ET AL: "Role of NSAIDs in acute treatment of headache", DRUG DEVELOPMENT RESEARCH, vol. 68, no. 6, January 2007 (2007-01), pages 276-281, XP055110059, ISSN: 0272-4391, DOI: 10.1002/ddr.20191

## Description

### BACKGROUND OF THE INVENTION

This invention relates to compositions for use in a method for treating or preventing pain associated with migraine headache. The method involves topical application of at least one therapeutic agent comprising ketoprofen in an amount of 1% to 30% (w/w) to the sternocleidomastoid muscle at the temporomandibular joint.

Migraine is a debilitating condition that is usually characterized by a throbbing pain on one or both sides of the head often accompanied by other neurological symptoms, such as visual disturbances, nausea, vomiting, dizziness, extreme sensitivity to sound, light, touch and smell, and tingling or numbness in the extremities or face. Migraine attacks, which typically last between 4 and 72 hours, vary in severity from person to person, and are believed to affect over 10% of the population worldwide. In the U.S. alone, it is estimated that 1 in 4 households includes someone who suffers from migraines.

Migraine headaches are typically the result of specific cerebral vasodilatations that press on and activate certain afferent pain receptors and pain receptor neural networks. Vasodilatation can be caused by passive means, whereby an artery loses vascular tone for a period of time (idiopathic vasodilatation) or as a result of inflammation induced vasodilatation. Behavior, food, and social factors can also play a role in the frequency and severity of a migraine headache.

For many years, ergot alkaloids, such as ergotamine and dihydroergotamine, were used to treat migraine headaches and cluster headaches. These agents, while marginally effective, had many serious and limiting side effects. More recently, drugs from a chemical class called triptans have been used to treat migraines. One of the first drugs in this class was sumatriptan, developed by Glaxo (now Glaxo Smith Kline). Triptans have a complex mechanism that involves serotonin receptor partial agonism of the 5HT (1B) and 5HT (1D) receptors. In humans, blood is shunted from a vasodilated region to another region of the cerebrovasculature, a lessening in vascular pressure occurs, which can ease an established headache. Triptans, as illustrated by sumatriptan, have been approved for oral or nasal administration but not for local application. Relapse rates vary among patients and, in some cases, patients experience a vascular coronary vasoconstriction or coronary vasospasm because of the excessively high plasma concentration. In patients with coronary artery disease, vasoconstriction is more likely because of endothelial dysfunction arising from lowered nitric oxide activity.

The dilation of the meningeal blood vessels increases the activity of the nerve endings of the primary afferent neurons of the trigeminal nerve that are wrapped around them. As a result, the trigeminal cells are believed to release calcitonin gene related protein (CGRP), a vasodilator neuropeptide, which further increases the dilation of the meningeal blood vessels, and further feeds into the trigeminal nerve activation. This local activation of the trigeminal nerve spreads through the trigeminal ganglion into the Trigeminal Nucleus Caudalis (TNC) in the brain stem in a process known as peripheral sensitization. The activation of the TNC leads in turn to a central activation process through its thalamic and cortical projections.

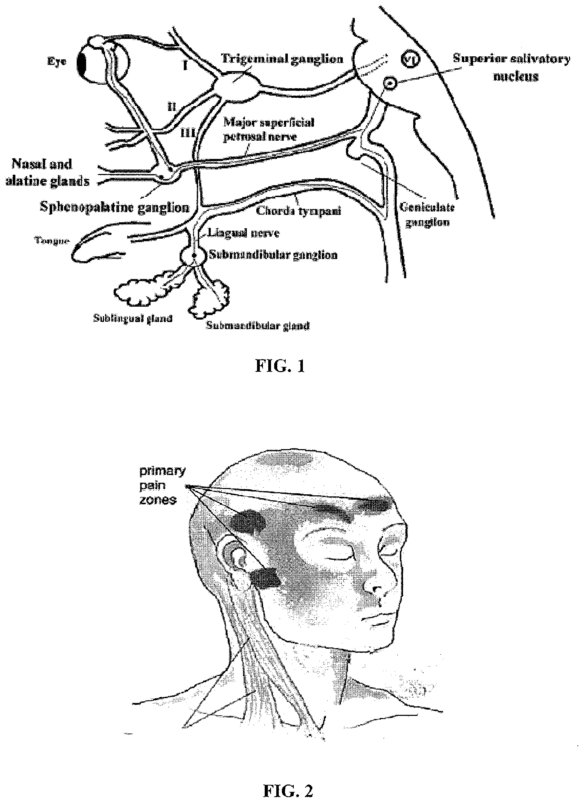

Although the pain associated with migraine involves input from meningeal arteries, activation of the TNC may result in referred pain anywhere along the trigeminal network, including the temporal arteries and temporal muscles. The trigemino-cervical network involved in the pathophysiology of migraine contains the three main branches of the trigeminal nerve: the ophthalmic branch (VI), the maxillary branch (V2), and the mandibular branch (V3) (see Figure 1), as well as the sensory nerves for the posterior head and neck (C2, C3, C4, C5) that feed into the TNC. A detailed anatomical map of the relevant pathways can be found in pages 316, 317, 600, 601 and 736 of Agur, A. M. R. and Dalley II, A. F. (2005) Atlas of Anatomy Ed., Lippincott Williams & Wilkins, Philadelphia.

The activation of the TNC in the brainstem can further spread to the occipital nerve by virtue of its anatomical connection to the TNC, leading to pain sensation in the occipital area and the observed vision disturbances associated with migraines. The activation of the TNC can also spread to the parasympathetic system, by activation of a nearby nucleus in the brain stem, the Superior Salivatory Nucleus (SSN), which is connected to the nucleus caudalis through a network of inter-neurons.

Neurons from the SSN synapse with the Sphenopalatine ganglion and provide vasomotor innervation to blood vessels and secretomotor innervation to the lacrimal glands, as well as the nasal and sinus mucosa. When the parasympathetic system is activated, the upper respiratory tract symptoms associated with migraine occur, including, potentially, nasal symptoms (rhinorrhea, and post nasal drip), ocular symptoms (conjunctival injection, and tearing), and sinus congestion (pain or pressure around the sinuses). Other parasympathetic projections further aggravate the cascade of events, like the Sphenopalatine ganglion afferents that innervate the meningeal blood vessels. Activation of the parasympathetic system during a migraine attack is also accompanied by a significant increase in the levels of Vasoactive Intestinal Polypeptide (VIP), a parasympathetic neurotransmitter which causes vasodilation and can be measured in high concentrations during a migraine in the jugular venous drainage.

The increased activity of the trigeminal, occipital and parasympathetic systems just described is common to the so-called Trigeminal Autonomic Cephalgias (TAC), which include Cluster headache, Paroxysmal Hemicrania, SUNCT Syndrome, and Hemicrania Continua.

Cluster headaches are a primary headache disorder involving attacks of less than 3 hours of duration with severe unilateral periorbital and temporal pain. These headaches can be associated with lacrimation, nasal congestion, rhinorrhea, conjunctival injection and a Homer's syndrome. The attacks occur in distinct clusters. Such cluster headaches typically involve a series of disabling attacks on a daily basis lasting for months at a time.

Paroxysmal hemicrania is a primary headache disorder involving frequent attacks of unilateral, periorbital and temporal pain typically lasting less than 30 minutes. The pain can be associated with conjunctival injection, lacrimation, nasal congestion, rhinorrhea, ptosis and eyelid edema.

SUNCT Syndrome is a primary headache disorder characterized by multiple attacks of unilateral, periorbital and temporal pain typically lasting less than 2 minutes. The pain is associated with conjunctival injection, lacrimation, nasal congestion, rhinorrhea, and eyelid edema. This headache may be associated with trigeminal neuralgia.

Hemicrania Continua is a primary headache disorder characterized by a strictly unilateral headache responsive to Indomethacin. The pain is associated with conjunctival injection, lacrimation, nasal congestion, rhinorrhea, ptosis, and eyelid edema.

Very importantly, the trigeminal nerve is involved in the pain sensations for all of these headache types, as well as headaches triggered by other pathologies. For example, Temporal Arteritis involves inflammation of the temporal artery with painful palpable nodules along the artery. In addition to headache in the temporal area, Temporal Arteritis causes vision loss and jaw pain.

Headaches can also be associated with ischemic stroke. In a stroke, a lack of blood supply to brain tissue causes a sudden localized neurological deficit. In a large number of affected patients, occlusion of the arteries is due to the presence of atherosclerotic plaques in the arteries supplying the brain, for example, the carotid artery and the vertebral basilar artery

The atherosclerotic plaques are often associated with inflammation which further contributes to occlusion of the blood vessel. Nociceptive fibers stimulated by inflammatory mediators in infectious or allergic rhinitis can also activate the trigeminal brainstem nucleus and precipitate migraine.

TAC and Migraine are difficult to treat. Numerous medications have been used to prevent and treat Cluster and Migraine headaches, which include, amongst others: Propranolol, Timolol, Divalproex Sodium, Topiramate Verapamil, Triptans, Ergot Alkoloids, Opiods Amitriptyline and the non-steroidal anti-inflammatories Indomethacin, aspirin, diclofenac, ibuprofen, ketoprofen, or naproxen, and others. These medicines have numerous side effects and patients are poorly compliant with them.

In US 2003/0119892, topical NSAID administration to a keratinized skin site proximal to the pain associated with a headache pain is disclosed.

In US 6,221,377, diclofenac is used to treat migraine headache by application to the temples, over the frontal areas and/or upper nuchal and/or occipital areas.

There is a strong need in the medical field for safe and effective methods and compositions to rapidly treat or prevent TAC, migraine headaches, tension headaches, cluster headaches, and/or other headaches associated with vascular conditions.

### SUMMARY OF THE INVENTION

The present invention provides a composition comprising from 1% to 30% (w/w) of ketoprofen and a dermatologically acceptable carrier for use in a method of treating or preventing pain associated with a migraine headache, the method comprising topically administering to a mammal having pain associated with a migraine headache an effective amount of the composition to the sternocleidomastoid muscle at the temporomandibular joint; wherein said composition is formulated as a cream, a gel, an ointment, or a liquid. The composition may comprise from 2.5% to 10% (w/w) ketoprofen and a dermatologically acceptable carrier. The composition may further comprise from 5% to 20% (w/w) gabapentin. The composition may comprise from 2% to 12% (w/w) ketoprofen and from 8% to 12% (w/w) gabapentin. Any of the compositions above may further comprise a skin penetrating enhancer, wherein said skin penetrating enhancer may be selected from the group consisting of isopropyl myristate, isopropyl palmitate, dimethyl sulfoxide, decyl methyl sulfoxide, dimethylalanine amide of a medium chain fatty acid, dodecyl 2-(N,N-dimethylamino) propionate or salts thereof, tetradecyl(N,N-dimethylamino) acetate, dodecyl(N,N-dimethylamino) acetate, decyl(N,N-dimethylamino) acetate, octyl(N,N-dimethylamino) acetate, and dodecyl(N,N-diethylamino) acetate.

Disclosed is a method for reducing or preventing symptoms, and in particular pain, related to migraines, TAC, and other headaches associated with vascular conditions in mammals, particularly humans, by using a method to topically administer anti-inflammatory agents. Specifically, disclosed is the topical administration of an effective amount of a therapeutic agent along the extracranial trigeminal nerve endings in the temporal region, the extracranial occipital nerve endings in the occipital region, and/or along the intranasal trigeminal nerve endings and/or the parasympathetic nerve endings in the nasal mucosa of a mammal.

Disclosed is a method of delivering a therapeutic agent to a subject, the method including topically administering to the orbital foramen region, the extracranial trigeminal nerve endings in the temporal region, the extracranial occipital nerve endings in the occipital region, and/or along the intranasal trigeminal nerve endings and/or the parasympathetic nerve endings in the nasal mucosa of the subject a composition including an effective amount of at least one therapeutic agent. The effective amount may be an amount effective to treat a headache (e.g., a migraine headache, a tension type headache, or a cluster headache). The orbital foramen region may include one or more of the supraorbital foramen, the base of an auriculo-temporal branch of the trigeminal nerve, the auriculo-temporal branch of a greater occipital nerve, the postauricular area, the forehead, the neck, or the side of the head.

The therapeutic agent may be selected from the group consisting of an alpha adrenoceptor agonist (e.g., phenylephrine, pseudoephedrine, or oxymetazoline), an anesthetic (e.g., physostigmine, neostigmine, or procaine), an anticonvulsant (e.g., gabapentin, topiramate, hydantoin, a benzodiazepine, zonisamide, valproic acid, ethosuximide, carbamazepine, primidone, lamotrigine, felbamate, levetiracetam, or tiagabine), an anticholinergic compound (e.g., ipratropium bromide, oxitropium bromide, or tiotropium), an antihistamine (e.g., carbinoxamine, clemastine, dimenhydrinate, pyrilamine, tripelennamine, chlorpheniramine, brompheniramine, hydroxyzine, cyclizine, acrivastine, cetririzine, azelastine, loratadine, fexofenadine, doxepin, diphenhydramine, amitriptyline, imipramine, promethazine, chlorpromazine, or nortriptyline), an antiinflammatory compound (e.g., aspirin, diclofenac, ibuprofen, ketoprofen, or naproxen; or cyclooxygenase inhibitor), a beta receptor antagonist (e.g., propranolol, nadolol, timolol, pindolol, labetalol, metroprolol, atenalol, esmolol, or acebutolol), an ion channel blocking compound (e.g., flunarizine, verapamil, nifedipine, or nimodipine), a N-methyl d-aspartate receptor agonist (e.g., dextromethorphan, ketamine, memantine, riluzole, or phencyclidine), a norepinephrine reuptake inhibitor (e.g., reboxetine, duloxetine, or amitriptyline), an opioid (e.g., morphine, codeine, meperidine, or oxycodone), a selective serotonin reuptake inhibitor, a serotonin agonist, serotonin partial agonist, and a triptan (e.g., almotriptan, eletriptan, frovatriptan, naratriptan, rizatriptan, sumatriptan, or zolmitriptanThe therapeutic agent may be selected from sumatriptan, ibuprofen, ketoprofen, diclofenac, dextromethorphan, gabapentin, amitriptyline, diphenhydramine, and doxepin. Ketoprofen and possibly gabapentin are comprised by the composition for use according to the invention.

The antiinflammatory compound (e.g., a cyclooxygenase inhibitor) may be administered together with at least one therapeutic agent selected from the group consisting of an anticonvulsant, an antihistamine, an ion channel blocking compound, a N-methyl d-aspartate receptor agonist, an opioid, and a triptan (e.g., the cyclooxygenase inhibitor is administered together with the ion channel blocking compound or the cyclooxygenase inhibitor is administered together with the antihistamine). Ketoprofen or ibuprofen may be administered together with gabapentin or topomirate; ketoprofen or ibuprofen may be administered together with diphenhydramine or dextromethorphan; ketoprofen or ibuprofen may be administered together with diphenhydramine and dextromethorphan; or ketoprofen or ibuprofen may be administered together with sumatriptan.

The antihistamine may be administered together with at least one therapeutic agent selected from the group consisting of an antiinflammatory compound, an anticonvulsant, an ion channel blocking compound, a N-methyl d-aspartate receptor agonist, an opioid, and a triptan (e.g., diphenhydramine is administered together with dextromethorphan).

The triptan may be administered together with at least one therapeutic agent selected from the group consisting of an antiinflammatory compound, an anticonvulsant, an antihistamine, an ion channel blocking compound, a N-methyl d-aspartate receptor agonist, and an opioid (e.g., sumatriptan is administered together with gabapentin; or sumatriptan is administered together with the antihistamine).

The composition for use according to the invention features a composition formulated for topical administration including from 1% to 30% (w/w) of ketoprofen and a dermatologically acceptable carrier.

The composition including from 1% to 30% (w/w) of ketoprofen may further include at least one therapeutic agent selected from the group consisting of an alpha adrenoceptor agonist, an anesthetic, an anticonvulsant (e.g., 5% to 20% (w/w) of gabapentin), an anticholinergic compound, an antihistamine, a beta receptor antagonist, an ion channel blocking compound, a N-methyl d-aspartate receptor agonist, a norepinephrine reuptake inhibitor, an opioid, a serotonin agonist, serotonin partial agonist, a selective serotonin reuptake inhibitor, and a triptan. The composition may include from 8% to 12% (w/w) of ketoprofen and from 8% to 12% (w/w) of gabapentin.

The composition including from 1% to 30% (w/w) of ketoprofen may further include a skin penetrating enhancer (e.g., isopropyl myristate, isopropyl palmitate, dimethyl sulfoxide, decyl methyl sulfoxide, dimethylalanine amide of a medium chain fatty acid, dodecyl 2-(N,N-dimethylamino) propionate or salts thereof, tetradecyl (N,N-dimethylamino) acetate, dodecyl (N,N-dimethylamino) acetate, decyl (N,N-dimethylamino) acetate, octyl (N,N-dimethylamino) acetate, and dodecyl (N,N-diethylamino) acetate).

The composition for use according to the invention features a composition including from 1% to 30% (w/w) of ketoprofen formulated as a cream, a gel, an ointment, or a liquid.

Disclosed is a composition formulated for topical administration including from 10% to 30% (w/w) of ibuprofen and a dermatologically acceptable carrier.

Tthe composition may further include at least one therapeutic agent selected from the group consisting of an alpha adrenoceptor agonist, an anesthetic, an anticonvulsant, an anticholinergic compound, an antihistamine, a beta receptor antagonist, an ion channel blocking compound, a N-methyl d-aspartate receptor agonist, a norepinephrine reuptake inhibitor, a serotonin agonist, serotonin partial agonist, a selective serotonin reuptake inhibitor, and a triptan. The therapeutic agent may be the antihistamine, the antihistamine and the N-methyl d-aspartate receptor agonist, or the triptan.

The composition may include from 1% to 5% (w/w) of dextromethorphan and from 1% to 5% (w/w) of diphenhydramine. The composition may include 20% (w/w) of ibuprofen, 2.5% (w/w) of dextromethorphan, and 2.5% (w/w) of diphenhydramine.

The composition including at least one therapeutic agent above may further include a skin penetrating enhancer (e.g., isopropyl myristate, isopropyl palmitate, dimethyl sulfoxide, decyl methyl sulfoxide, dimethylalanine amide of a medium chain fatty acid, dodecyl 2-(N,N-dimethylamino) propionate or salts thereof, tetradecyl (N,N-dimethylamino) acetate, dodecyl (N,N-dimethylamino) acetate, decyl (N,N-dimethylamino) acetate, octyl (N,N-dimethylamino) acetate, and dodecyl (N,N-diethylamino) acetate).

The composition including at least one therapeutic agent above may be formulated as a cream, a gel, an ointment, or a liquid.

### Definitions

As used herein, the term "administration" or "administering" refers to a method of giving a dosage of a pharmaceutical composition to a subject. The preferred method of administration may depend on a variety of factors, e.g., the components of the pharmaceutical composition and the nature and severity of the disease, disorder, or condition. The phrase "administered together" means that two or more therapeutic agents are formulated together in a single pharmaceutical composition that is administered to the subject.

As used herein, the phrase "an amount effective to treat a headache" refers to an amount of at least one therapeutic agent that prevents the headache, diminishes the frequency or intensity of the headache, or relieves one or more symptoms caused by the headache.

As used herein, the phrase "an effective amount" refers to an amount of at least one therapeutic agent that prevents, treats, or palliates a disease, a disorder, or a condition.

The phrase "dermatologically acceptable" means that the compositions or components thereof are suitable for use in contact with dermal tissue without undue toxicity, incompatibility, instability, allergic response, and the like.

By "headache" is meant any type of headache, including, but not limited to, a migraine headache, a tension headache, or a cluster headache.

By "orbital foramen region" is meant an area of the subject including the supraorbital foramen and surrounding regions, such as the base of the auriculo-temporal branch of the trigeminal nerve or the auriculo-temporal branch of the greater occipital nerve, the postauricular area, the forehead, including either side of the forehead, or the neck.

By "subject" is meant a mammal, including, but not limited to, a human or non-human mammal.

By "treatment" is meant an approach for obtaining beneficial or desired results, such as clinical results. Beneficial or desired results can include, but are not limited to, alleviation, amelioration, or prevention of a disease, a disorder, a condition, or one or more symptoms associated with a disease, a disorder, or a condition; diminishment of extent of disease, disorder, or condition; stabilization (i.e., not worsening) of a disease, disorder, or condition; delay or slowing the progress of a disease, disorder, or condition; and amelioration or palliation of a disease, disorder, or condition. "Treatment" can also mean prolonging survival as compared to expected survival if not receiving treatment. By "prevention" is meant that a prophylactic treatment is given to a subject who has or will have a disease, a disorder, a condition, or one or more symptoms associated with a disease, a disorder, or a condition.

By "palliation" of a disease, a disorder, or a condition is meant that the extent and/or undesirable clinical manifestations of the disease, disorder, or condition are lessened and/or the time course of the progression is slowed or lengthened, as compared to the extent or time course in the absence of treatment.

The recitation herein of numerical ranges by endpoints is intended to include all numbers subsumed within that range (e.g. a recitation of 1 to 5 includes 1, 1.5, 2, 2.75, 3, 3.80, 4, and 5).

As used herein, "a" or "an" means "at least one" or "one or more" unless otherwise indicated. In addition, the singular forms "a", "an", and "the" include plural referents unless the context clearly dictates otherwise. Thus, for example, reference to a composition containing "a therapeutic agent" includes a mixture of two or more therapeutic agents.

Other features and advantages of the invention will be apparent from the following Detailed Description and from the claims.

### BRIEF DESCRIPTION OF THE DRAWINGS

Figure 1 is a diagram of the trigeminal and parasympathetic network involved in headache pain, from and into the brainstem.
Figure 2 is a diagram of areas of application to treat trigeminal cephalgias, such as migraine and migraine-like headaches and other cerbrovascular conditions associated with pain and/or inflammation.
Figure 3 is a graph showing pain level over time (on a scale from 1 to 8 where 8 is the most severe pain) in subjects treated with a ketoprofen formulation. The drug was administered 30-45 minutes after onset of pain.
Figure 4 is a graph showing pain level over time (on a scale from 1 to 8 where 8 is the most severe pain) in a subject suffering from multiple migraine attacks treated with a ketoprofen formulation. The drug was administered 30-45 minutes after onset of pain.
Figure 5 is a graphical respresentation of the pain intensity level on a scale from 1 to 8 where 8 is the most severe pain in one patient with multiple migrane-like headaches. The drug was applied 30 minutes after onset of initial pain and upon return of subsequent pain.

### DETAILED DESCRIPTION

Disclosed is a method for treatment and/or prevention of symptoms, especially pain, related to migraines, TAC, and other headaches associated with vascular conditions in mammals, particularly humans. Disclosed is such method to treat and prevent these headaches using presynaptic neuromodulators. Without being bound to any particular theory, it is believed that blockade of the release of nociceptive and inflammatory agents triggered by the hyperactivation of the trigeminal, occipital and parasympathetic systems involved in the development of these headaches provides an effective therapeutic and/or prophylactic treatment. The anatomical pathways involved in the development of these headaches are intracranial; therefore, specific blockade of the intracranial pathways involved by non-invasive means is not feasible. However, some of the pathways affected are located extracranially under the surface of the skin and are therefore accessible to treatment. Accordingly, the method involves administering a therapeutically effective amount of a pharmaceutically safe presynaptic neuromodulator, peripherally, along the extracranial trigeminal nerve endings in the temporal region, the extracranial occipital nerve endings in the occipital region, and/or along the intranasal trigeminal nerve endings and/or the parasympathetic nerve endings in the nasal mucosa of a mammal.

Disclosed is a method for treating headaches, including symptoms associated with headaches. The method involves topical administration of at least one therapeutic agent to the orbital foramen region of the patient. The orbital foramen region in mammals includes the notch of the eyebrow (or supraorbital foramen), which is an area where drugs in liquid formulation or as an ointment or as a cream formulation can penetrate into the regional arterial space and affect cerebral blood flow. The orbital foramen region also includes areas surrounding the supraorbital foramen, such as the base of the auriculo-temporal branch of the trigeminal nerve or the auriculo-temporal branch of the greater occipital nerve. These compositions may also be administered to the postauricular area, such as the area back of the ear; the forehead; or the either side of the head, such as on the left or right side of the scalp just above the ears, in the form of a liquid, ointment, lotion or patch.

Also disclosed herein are dermatologically acceptable compositions for treating headaches. We have determined that migraine pain immediately ceases (e.g., within 15-30 minutes) when a composition containing a suitable therapeutic agent, such as a cyclooxygenase inhibitor, either alone or in combination with other therapeutic agents, is applied to the eyebrow notch of a subject. Upon application, the preferred composition blocks pain completely within 30 minutes. The response rate with the most effective combinations blocked pain by over 70% in a diverse set of individuals.

The methods and compositions disclosed herein or the compositions of the invention can be used to treat a headache either by preventing the recurrence of a headache or by treating one or more symptoms associated with an established headache. The methods and compositions disclosed herein or or the compositions of the invention can be used to treat any type of headache, including a migraine headache, a tension headache, or a cluster headache. However, according to the invention, the compositions for use according to the invention are for use in a method for treating or preventing pain associated with migraine headache. Symptoms and diagnosis of headaches can be readily determined by any parameters well known in the art, for example, as described in "The International Classification of Headache Disorders" (2d ed., ed. International Headache Society).

Exemplary symptoms of a migraine headache include: moderate to severe headache intensity; a unilateral headache; a headache with a pulsating or throbbing quality; a headache that worsens with physical activity; a headache that interferes with regular activities; nausea; vomiting; sensitivity to light, sound, or smell; depression; sleep disruption; ptosis; experiencing an aura, such as changes to vision; or paresthesia of the hand, arm, leg, or face.

The signs and symptoms of migraine vary among patients. Therefore, what a patient experiences before, during, and after an attack cannot be precisely defined. The four common phases of a migraine attack are listed below. However, the phases experienced and the symptoms experienced during those phases can vary from one migraine attack to another in the same migraineur (or migraine sufferer). These phases include: the prodrome phase, which occurs hours or days before the headache; the aura phase, which immediately precedes the headache; the pain phase, also known as headache phase; and the postdromal phase.

Symptoms within the prodromal phase include altered mood, irritability, depression or euphoria, fatigue, yawning, excessive sleepiness, craving for certain food (e.g., chocolate), stiff muscles (e.g., in the neck), constipation or diarrhea, increased urination, and other visceral symptoms. These symptoms usually precede the headache phase of the migraine attack by several hours or days, and experience teaches the patient or observant family how to detect that a migraine attack is near.

The aura phase comprises focal neurological phenomena that precede or accompany the attack. This phase typically appears gradually over 5 to 20 minutes and generally last fewer than 60 minutes. Symptoms of migraine aura can be visual, sensory, or motor in nature. Neurological symptoms during the visual aura phase include disturbance of vision (e.g., photopsia or scintillating scotoma, which is called "fortification spectra" or "teichopsia"); blurred or shimmering or cloudy vision; tunnel vision; and hemianopsia. Symptoms during the somatosensory aura phases include digitolingual or cheiro-oral paresthesias in the hand, in the arm, on the nose-mouth area on the same side; or on the face, lips and tongue. Other symptoms of the aura phase can include auditory or olfactory hallucinations, temporary dysphasia, vertigo, tingling or numbness of the face and extremities, and hypersensitivity to touch.

The pain phase usually begins within 60 minutes of the end of the aura phase, but can be delayed up to several hours, and it can be missing entirely. The typical migraine headache is unilateral, throbbing, and moderate to severe and can be aggravated by physical activity. Not all these features are necessary. The pain may be bilateral at the onset or start on one side and become generalized, and usually it alternates sides from one attack to the next. The onset is usually gradual. The pain peaks and then subsides and usually lasts 4 to 72 hours in adults and 1 to 48 hours in children. The frequency of attacks is extremely variable, from a few in a lifetime to several times a week, where the average migraineur experiences one to three headaches a month. The head pain varies greatly in intensity.

The pain of migraine is invariably accompanied by other features. Nausea occurs in almost 90 percent of patients, and vomiting occurs in about one third of patients. Many patients experience sensory hyperexcitability manifested by photophobia, phonophobia, and osmophobia, where these patients typically seek a dark and quiet room. Typical symptoms include: blurred vision, nasal stuffiness, diarrhea, polyuria, pallor, sweating, localized edema of the scalp or face, scalp tenderness, prominence of a vein or artery in the temple, stiffness and tenderness of the neck, or impairment of concentration and mood are common. The extremities tend to feel cold and moist. Vertigo may be experienced; a variation of the typical migraine, called vestibular migraine, has also been described. Lightheadedness, rather than true vertigo, and a feeling of faintness may occur.

In the postdromal phase, the patient may feel tired or "hung-over" and have head pain, cognitive difficulties, gastrointestinal symptoms, mood changes, and weakness. Some people feel unusually refreshed or euphoric after an attack, whereas others note depression and malaise. Often, some of the minor headache phase symptoms may continue, such as loss of appetite, photophobia, and lightheadedness. For some patients, a 5- to 6-hour nap may reduce the pain, but slight headaches may still occur when the patient stands or sits quickly. These symptoms may go away after a good night's rest, although there is no guarantee. Some people may suffer and recover differently than others.

Tension type headaches (TTH) include those that are episodic or chronic. Both types of tension type headaches exhibit similar symptoms, where chronic TTH typically evolves from an episodic TTH. A chronic TTH typically lasts >15 days per month, where an episodic TTH typically lasts <15 days per month. Exemplary symptoms of chronic and episodic TTH include: headaches lasting from 30 minutes to 7 days; a nonpulsating feeling, such as pressing or tightening; mild or moderate intensity; bilateral location; lack of aggravation from routine physical activity; lack of nausea or vomiting; photophobia; or phonophobia.

A cluster headache is characterized as a trigeminal autonomic cephalgia. Exemplary symptoms include: severe or very severe unilateral orbital, supraorbital and/or temporal pain; ipsilateral conjunctival injection and/or lacrimation; ipsilateral nasal congestion and/or rhinorrhoea; ipsilateral eyelid edema; ipsilateral forehead and facial sweating; ipsilateral miosis and/or ptosis; or a sense of restlessness or agitation.

### Therapeutic agents

Suitable therapeutic agents or combinations thereof for use in the compositions and methods generally include those that will act locally to decrease painful vasodilatation of superficial cerebral arteries. For example, a therapeutic agent or combination thereof that provides an antihistaminic effect may do so in any number of ways, such as by H-1 receptor antagonism, by preventing mast cell degranulation, or by the release of histamine contained in mast cells that reside in the vicinity of the superficial cerebral arteries. Suitable therapeutic agents or combinations thereof also include, but are not limited to, those that inhibit the re-uptake of norepinephrine from the nerve endings that surround the vasodilated cerebral artery; exhibit anti-cholinergic activity; provide local anesthetic activity; or have specific ion channel blocking activity, such as blocking sodium uptake and/or decrease the afferent activity of the nervous system or act functionally as non-steroidal anti-inflammatory drugs (by incorporation all drugs referred to as NSAIDS).

The methods and compositions typically utilize a therapeutic agent, either alone or in combination. Exemplary classes of therapeutic agents that may be used in the methods and/or compositions include an alpha adrenoceptor agonist; an anesthetic; an anticonvulsant; an anticholinergic compound; an antihistamine, including a tricyclic with antihistaminic activity; an antiinflammatory compound, such as a cyclooxygenase (COX) inhibitor or a non-steroidal antiinflammatory drug (NSAID); a beta receptor antagonist; an ion channel blocking compound, such as a sodium channel blocker or a calcium channel blocker; a N-methyl d-aspartate (NMDA) receptor antagonist; a norepinephrine reuptake inhibitor; an opioid; a selective serotonin reuptake inhibitor; a serotonin agonist; a serotonin partial agonist; and/or a triptan. Therapeutic agents to be formulated alone or in combination may include: sumatriptan, ibuprofen, ketoprofen, diclofenac, dextromethorphan, gabapentin, amitriptyline, diphenhydramine, and doxepin.

Examples of alpha adrenoceptor agonists include phenylephrine, pseudoephedrine, and oxymetazoline. Examples of anesthetics include physostigmine, neostigmine, and procaine. Examples of anticonvulsants include gabapentin, topiramate, hydantoin, benzodiazepines, zonisamide, valproic acid, ethosuximide, carbamazepine, primidone, lamotrigine, felbamate, levetiracetam, and tiagabine. Examples of anticholinergic compounds include ipratropium bromide, oxitropium bromide, or tiotropium. Examples of antihistamines include carbinoxamine, clemastine, dimenhydrinate, pyrilamine, tripelennamine, chlorpheniramine, brompheniramine, hydroxyzine, cyclizine, acrivastine, cetririzine, azelastine, loratadine, fexofenadine, doxepin, diphenhydramine, and all tricyclics that have antihistaminic activity, such as amitriptyline, imipramine, promethazine, chlorpromazine, and nortriptyline.

Examples of antiinflammatory compounds include aspirin, diclofenac, and COX inhibitors. Exemplary COX inhibitors include a non-selective COX inhibitor, such as an inhibitor for COX-1 and COX-2 or an inhibitor for COX and lipoxygenase (LOX); a selective COX-1 inhibitor; a selective COX-2 inhibitor; and/or a selective COX-3 inhibitor. Examples of COX inhibitors include ibuprofen, including a racemic mixture or an enantiomer thereof; ketoprofen, including a racemic mixture or an enantiomer thereof; and/or naproxen.

Examples of beta receptor antagonists include propranolol, nadolol, timolol, pindolol, labetalol, metroprolol, atenalol, esmolol, and acebutolol. Examples of ion channel blocking compounds include flunarizine, verapamil, nifedipine, and nimodipine. Examples of NMDA receptor antagonists include dextromethorphan, ketamine, memantine, riluzole, and phencyclidine. Examples of norepinephrine reuptake inhibitor include reboxetine, duloxetine, and amitriptyline. Examples of opioids include morphine, codeine, meperidine, and oxycodone. Examples of triptans include almotriptan, eletriptan, frovatriptan, naratriptan, rizatriptan, sumatriptan, and zolmitriptan.

Regarding the composition for use according to the invention or where the composition disclosed herein is applied topically to the patient, the therapeutic agents used in the composition should have appropriate properties for topical administration. For example, suitable therapeutic agents for topical formulations include those that will act locally and upon absorption will be diluted into the large blood volume of the vascular space; or produce no adverse events. Suitable therapeutic agents and combinations are best administered in a non-greasy ointment or in a cream base.

Combinations of two or more therapeutic agents can be administered to a subject to treat a migraine headache. Exemplary combinations include a combination of an antiinflammatory compound and an ion channel blocking compound, such as a COX-2 inhibitor and a calcium channel blocking compound; a combination of a COX inhibitor and an antihistamine; a combination of a general antiinflammatory compound and an NMDA receptor antagonist; a combination of a triptan and a general antiinflammatory compound; a combination of a triptan and an antihistamine; and a combination of a general antiinflammatory compound and an opioid. In general, the following combinations would be useful for a migraine: muscle relaxants, such as anticonvulsants; opioids; analgesics, such as narcotic analgesics, opioids, NSAIDs, or COX inhibitors; NSAIDs; serotonergic agonists, such as a serotonin agonist or a serotonin partial agonist; COX-2 inhibitors; nitrates; beta blockers or beta receptor antagonist; anticonvulsants, such as hydantoin, benzodiazepines, or topiramate; alpha agonists or alpha adrenoceptor agonists; antihistamines; and local anesthetics.

### Dosage, Formulation, and Administration

The compositions for use according to the invention or the compositions disclosed herein may conveniently be administered in unit dosage form and may be prepared by any of the methods well-known in the pharmaceutical art, for example, as described in "Remington: The Science and Practice of Pharmacy" (20th ed., ed. A. R. Gennaro, 2000, Lippincott Williams & Wilkins). The concentration of at least one therapeutic agent in the formulation will vary depending upon a number of factors, including the dosage of the drug to be administered, and the route of administration.

The therapeutic agents may be optionally administered as a pharmaceutically acceptable salt, such as a non-toxic acid addition salts or metal complexes that are commonly used in the pharmaceutical industry. Examples of acid addition salts include organic acids such as acetic, lactic, pamoic, maleic, citric, malic, ascorbic, succinic, benzoic, palmitic, suberic, salicylic, tartaric, methanesulfonic, toluenesulfonic, or trifluoroacetic acids or the like; polymeric acids such as tannic acid, carboxymethyl cellulose, or the like; and inorganic acid such as hydrochloric acid, hydrobromic acid, sulfuric acid phosphoric acid, or the like. Metal complexes include zinc, iron, and the like.

The composition can be prepared by any useful method. For example, at least one therapeutic agent is dissolved in ethanol and added to a mixture of polyethylene glycols (PEGs). In another example, the composition further includes a skin penetrating enhancer of a dimethyl alanine amide of medium chain fatty acids with carbon units varying between C-12 and C-16. More specifically, therapeutic agents alone or combinations thereof may be prepared in an ointment form or a cream form. In these forms, unit dispensing would be preferred, where the unit dosage of the therapeutic agent and vehicle would be in the range of 100 mg to 1000 g and most preferred between 100 mg and 500 mg. The therapeutic agent in this composition by weight would be in the range of 1% to 30 % (w/w). The most preferred range would be between 5% and 10% (w/w). In another embodiment, the composition comprises between 1%-2%, 2.5%-5%, 8%-12%, 10%-20%, or 20-30% (w/w) of at least one therapeutic agent.

The therapeutic agent may be present in the composition in an amount of at least 1%, at least 2%, at least 2.5%, at least 3%, at least 5%, at least 10%, at least 15%, at least 20%, or at least 25% (w/w), and may be, for example, ketoprofen or ibuprofen.

The therapeutic agent comprised by the composition for use according to the invention comprises ketoprofen in an amount of 1% to 30% (w/w), preferably 2.5% to 10% (w/w), and possibly gabapentin in an amount of 5% to 20% (w/w), and still more preferably ketoprofen in an amount of 2% to 12% (w/w) and gabapentin in an amount from 8% to 12% (w/w).

Optimization of the appropriate dosages can readily be made by the skilled practitioner in light of the pharmacokinetics of the therapeutic agent or combination of therapeutic agents used in the composition. Factors to be considered in setting dosages include the therapeutic agent's specific activity; the severity of the condition or symptoms of the subject; the age, condition, body weight, sex, and diet of the subject; the use (or not) of concomitant therapies; and other clinical factors.

Administration may be one or multiple times daily, weekly (or at some other multiple day interval) or on an intermittent schedule, with that cycle repeated a given number of times (e.g., 2-10 cycles) or indefinitely. Alternatively, the compositions may be administered as symptoms occur.

The compositions are typically administered as soon as possible after a headache begins. The composition may comprise between 1% to 30% (w/w) of at least one therapeutic agent (e.g., ketoprofen; gabapentin; dextromethorphan; a multi-mechanistic tricyclic molecule with activities including neurotransmitter uptake inhibition, antihistaminic or anticholinergic activity, such as imipramine, amitriptyline, and nortriptyline; or combinations thereof), whereby the composition for use according to the invention comprises ketoprofen in an amount of 1% to 30% (w/w) and possibly gabapentin in an amount of 5% to 20% (w/w).

The compositions can be formulated using any dermatologically acceptable carrier. Exemplary carriers include a solid carrier, such as alumina, clay, microcrystalline cellulose, silica, or talc; and/or a liquid carrier, such as an alcohol, a glycol, or a water-alcohol/glycol blend. The therapeutic agents may also be administered in liposomal formulations that allow therapeutic agents to enter the skin. Such liposomal formulations are described in U.S. Pat. Nos. 5,169,637; 5,000,958; 5,049,388; 4,975,282; 5,194,266; 5,023,087; 5,688,525; 5,874,104; 5,409,704; 5,552,155; 5,356,633; 5,032,582; 4,994,213; and PCT Publication No. WO 96/40061. Examples of other appropriate vehicles are described in U.S. Pat. No. 4,877,805 and EP Publication No. 0586106A1. Suitable vehicles may also include mineral oil, petrolatum, polydecene, stearic acid, isopropyl myristate, polyoxyl 40 stearate, stearyl alcohol, or vegetable oil.

The composition can further include a skin penetrating enhancer, such as those described in "Percutaneous Penetration enhancers", (eds. Smith EW and Maibach HI. CRC Press 1995). Exemplary skin penetrating enhancers include alkyl (N,N-disubstituted amino alkanoate) esters, such as dodecyl 2-(N,N dimethylamino) propionate (DDAIP), which is described in patent U.S. Pat. Nos. 6,083,996 and 6,118,020; a water-dispersible acid polymer, such as a polyacrylic acid polymer, a carbomer (e.g., Carbopol™ or Carbopol 940P™, available from B. F. Goodrich Company (Akron, Ohio)), copolymers of polyacrylic acid (e.g., Pemulen™ from B. F. Goodrich Company or Polycarbophil™ from A. H. Robbins, Richmond, Va.; a polysaccharide gum, such as agar gum, alginate, carrageenan gum, ghatti gum, karaya gum, kadaya gum, rhamsan gum, xanthan gum, and galactomannan gum (e.g., guar gum, carob gum, and locust bean gum), as well as other gums known in the art (see for instance, Industrial Gums: Polysaccharides & Their Derivatives, Whistler R. L., BeMiller J. N. (eds.), 3rd Ed. Academic Press (1992) and Davidson, R. L., Handbook of Water-Soluble Gums & Resins, McGraw-Hill, Inc., N.Y. (1980)); or combinations thereof.

Other suitable polymeric skin penetrating enhancers are cellulose derivatives, such as ethyl cellulose, methyl cellulose, hydroxypropyl cellulose. Additionally, known transdermal penetrating enhancers can also be added, if desired. Illustrative are dimethyl sulfoxide (DMSO) and dimethyl acetamide (DMA), 2-pyrrolidone, N,N-diethyl-m-toluamide (DEET), 1-dodecylazacycloheptane-2-one (Azone™, a registered trademark of Nelson Research), N,N-dimethylformamide, N-methyl-2-pyrrolidone, calcium thioglycolate and other enhancers such as dioxolanes, cyclic ketones, and their derivatives and so on.

Also illustrative are a group of biodegradable absorption enhancers which are alkyl N,N-2-(disubstituted amino) alkanoates as described in U.S. Pat. No. 4,980,378 and U.S. Pat. No. 5,082,866, including: tetradecyl (N,N-dimethylamino) acetate, dodecyl (N,N-dimethylamino) acetate, decyl (N,N-dimethylamino) acetate, octyl (N,N-dimethylamino) acetate, and dodecyl (N,N-diethylamino) acetate.

Particularly preferred skin penetrating enhancers include isopropyl myristate; isopropyl palmitate; dimethyl sulfoxide; decyl methyl sulfoxide; dimethylalanine amide of a medium chain fatty acid; dodecyl 2-(N,N-dimethylamino) propionate or salts thereof, such as its organic (e.g., hydrochloric, hydrobromic, sulfuric, phosphoric, and nitric acid addition salts) and inorganic salts (e.g., acetic, benzoic, salicylic, glycolic, succinic, nicotinic, tartaric, maleic, malic, pamoic, methanesulfonic, cyclohexanesulfamic, picric, and lactic acid addition salts), as described in U.S. Pat. No. 6,118,020; and alkyl 2-(N,N-disubstituted amino)-alkanoates, as described in U.S. Pat. No. 4,980,378 and U.S. Pat. No. 5,082,866.

The skin penetrating enhancer in this composition by weight would be in the range of 0.5% to 10 % (w/w). The most preferred range would be between 1.0% and 5% (w/w). In another embodiment, the skin penetrating enhancer comprises between 0.5% -1%, 1%-2%, 2%-3%, 3%-4%, or 4%-5%, (w/w) of the composition.

The compositions can be provided in any useful form. For example, the compositions disclosed herein may be formulated as solutions, emulsions (including microcmulsions), suspensions, creams, foams, lotions, gels, powders, or other typical solid, semi-solid, or liquid compositions used for application to the skin or other tissues where the compositions may be used. The compositions for use according to the invention are formulated as creams, gels, ointments, or liquids. The compositions may also be applied as a patch, preferably to the postauricular area or on the neck just behind the ear. Such compositions may contain other ingredients typically used in such products, such as colorants, fragrances, thickeners (e.g., xanthan gum, a fatty acid, a fatty acid salt or ester, a fatty alcohol, a modified cellulose, a modified mineral material, Krisgel 100™, or a synthetic polymer), antimicrobials, solvents, surfactants, detergents, gelling agents, antioxidants, fillers, dyestuffs, viscosity-controlling agents, preservatives, humectants, emollients (e.g., natural or synthetic oils, hydrocarbon oils, waxes, or silicones), hydration agents, chelating agents, demulcents, solubilizing excipients, adjuvants, dispersants, skin penetrating enhancers, plasticizing agents, preservatives, stabilizers, demulsifiers, wetting agents, sunscreens, emulsifiers, moisturizers, astringents, deodorants, and optionally including anesthetics, anti-itch actives, botanical extracts, conditioning agents, darkening or lightening agents, glitter, humectants, mica, minerals, polyphenols, silicones or derivatives thereof, sunblocks, vitamins, and phytomedicinals.

The compositions can also include other like ingredients to provide additional benefits and improve the feel and/or appearance of the topical formulation. Specific classes of additives commonly use in these formulations include: isopropyl myristate, sorbic acid NF powder, polyethylene glycol, phosphatidylcholine (including mixtures of phosphatidylcholine, such as phospholipon G), Krisgel 100™, distilled water, sodium hydroxide, decyl methyl sulfoxide (as a skin penetrating enhancer), menthol crystals, butylated hydroxytoluene, ethyl diglycol reagent, and 95% percent (190 proof) ethanol.

The compositions disclosed herein can be administered in any number of ways. The compositions for use according to the invention are topically administered. For example, the compositions in liquid form can be applied from absorbent pads; used to impregnate bandages and other dressings; or sprayed directly onto the orbital foramen or surrounding areas of the subject. In another example, the composition in solid form, including semi-solid form, can be applied from a tube; or be applied directly onto the orbital foramen or surrounding areas of the subject. In yet another example, the composition in liquid form or solid form can be applied by using an applicator to spread the composition onto the orbital foramen region. The composition may also be applied to the skin under occlusive dressing in a dermal delivery system (e.g., a transdermal patch).

A composition may be applied to the orbital foramen region as an ointment via an applicator containing the at least one therapeutic agent on a swab applicator. Alternatively, the composition may be applied as a lotion from a tube or tin. Furthermore, the composition may be applied as a lotion from a droptainer dispenser for larger volumes. Thereby, the composition may be applied using the fingers to rub the composition onto the region of the eyebrow notch. A transdermal patch with the composition or at least one therapeutic agent can also be used and applied, most preferably behind the ear.

Administration of therapeutic agents in controlled release formulations may be useful where the therapeutic agent has (i) a narrow therapeutic index (e.g., the difference between the plasma concentration leading to harmful side effects or toxic reactions and the plasma concentration leading to a therapeutic effect is small; (ii) a narrow slow absorption rate by or through the epithelium and/or dermis; or (iii) a short biological half-life, so that frequent dosing during a day is required in order to sustain a therapeutic level.

Many strategies can be pursued to obtain controlled release in which the rate of release outweighs the rate of metabolism of the therapeutic agent. For example, controlled release can be obtained by the appropriate selection of formulation parameters and ingredients, including, e.g., appropriate controlled release compositions and coatings. Examples include oil solutions, suspensions, emulsions, microcapsules, microspheres, nanoparticles, patches, and liposomes.

Further features and advantages of this invention are further illustrated by the following examples, which are in no way intended to be limiting thereof.

### EXAMPLES

### Example 1: Preparation of a cream base

To make 100 mL of a cream base, sorbic acid NF powder (0.3 g) was dissolved in isopropyl myristate NF (50 mL). To this mixture was added phospholipon G (45.45 g) with low heat or allowed to stand overnight. A liquid of syrup consistency formed, which should be shaken well. The cream base had a yellowish syrupy appearance. For less penetration efficiency, isopropyl myristate can be substituted with isopropyl palmitate. This cream base was used to formulate the therapeutic agents into final formulations, which is described in Example 4.

### Example 2: Preparation of an ointment base

To prepare 100 mL of an ointment base, decyl methyl sulfoxide (0.6 g) was crushed in a mortar and placed in glass bottle large enough to hold minimally 100 mL. Everclear 95% ethanol (19 mL) was added to the decyl methyl sulfoxide and shaken vigorously. Propylene glycol (58 mL) and ethoxy diglycol (20 mL) were added to this mixture and shaken together.

Menthol crystals (0.08 g) and butylated hydroxytoluene (0.05 g) were crushed in a mortar. A small amount (2 mL) of Everclear 95% ethanol was added to dissolve the crushed menthol crystals and butylated hydroxytoluene.

As a final step, the mixture of menthol and butylated hydroxytoluene was added to the decyl methyl sulfoxide mixture and shaken well. This final mixture was stored in a glass bottle, where this ointment can be used as base in the preparation of the therapeutic agents.

Furthermore, it is possible to add additional or different therapeutic agents or vary the percentage of the therapeutic agent in the cream or ointment. Sumatriptan alone may be used. Sumatriptan may also be used with an antihistamine. Sumatriptan may be used with ketoprofen or alternatively gabapentin may be used alone or in combination with sumatriptan or an antihistamine.

### Example 3: Preparation of a cream base

To make 100 mL of a cream base, Carbomer NF, Isopropyl Myristate, Disodium EDTA, Sodium MethylParaben, Sodium Propyl n Parabens were mixed using a suitable mixer as described in U.S. Pat. No. 6,083,996 to Biiyiiktimkin, et al., entitled "Topical Compositions For NSAID Drug Delivery" that discusses specific topical formulations for enhanced delivery of NSAID across human skin. This cream base was used to formulate the therapeutic agents into final formulations, which is described in Example 5.

### Example 4: Preparation of cream with therapeutic agents

The cream base detailed in Example 1 (10 g) was added to propylene glycol (5 mL) and dextromethorphan (5g). This mixture was blended together. Then, 10% sodium hydroxide (2.5 mL) was added to the mixture. Ibuprofen (or ketoprofen) (30 g) was added to the mixture. Krisgel 100™ (4 g, a thickener) and distilled water (46 g) was added, mixed thoroughly, and was adjusted to have a pH of about 5-6.5 with 10% sodium hydroxide. This cream was then packaged in ointment dispensing tubes.

### Example 5: Preparation of ointment with therapeutic agents

To prepare 50 g of drug product, 2.5 g of micronized ketoprofen were dissolved in 15 g of ethyl alcohol (95%) and mixed with the formulation of Example 3 (32.5g) with vigorous mixing (mix for at least 5 minutes under vigorous mixing). To the resulting product triethanolamine (0.75g) were added and pH adjusted to between 5.3 and 5.5,with drop-wise addition of triethanolamine, if needed, under vigorous mixing.

### Example 6: Patient study with ketoprofen and gabapentin

In this study, patients were given a combination of 10% ketoprofen and 10% gabapentin in an ointment (Example 2) to treat an established migraine headache. In this study, 90% were women and 10% were male. All patients were 18 years of age or older and consented to this study. In addition, 80% of these patients were in the age range of 45-60 and 20% are in the age range of 13-45.

The application of a combination of ketoprofen at 10% strength and gabapentin at 10% strength gave prompt (15 -30 minutes), safe, and effective relief of migraine. All patients involved in this protocol had had previous headaches that could be treated using standard methods. Relief of headache symptoms using standard therapy took an average 6 hours. Application of a mixture of ketoprofen (10%) and gabapentin (10%) provided immediate relieve in 70% of the patients within 30 minutes.

### Example 7: Patient studies with 5% ointment of ketoprofen

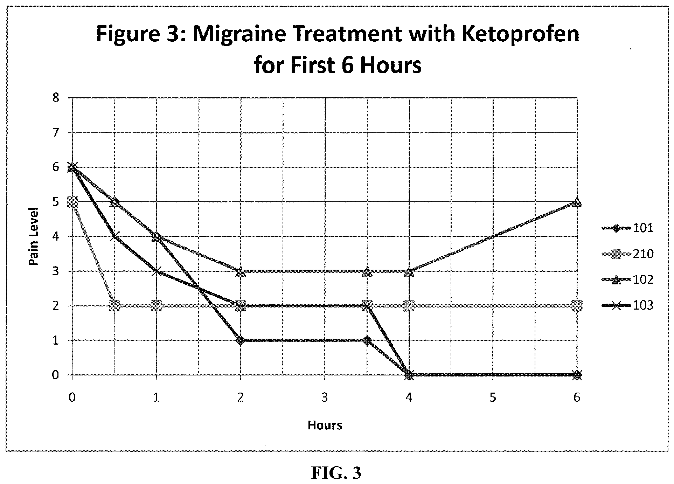

In this study, patients with an established migraine headache were given a 5% ointment formulation of ketoprofen as described in Example 5 above and instructed to apply to their forehead at the area of the eyebrow notch, behind the ear at the top of the jaw bone and to the temple area above the ear. Patients were asked to rate their pain on a scale of 1 to 8 where 8 is an unbearable pain and 1 is minimal pain. If pain persisted or returned after a single application of ketoprofen patients were instructed to reapply the medication up to 4 times / day. The results are provided in Figures 3-5.

Figure 3 illustrates results with four patients (patient number 101, 102, 103, and 210) with established migraines. This figure shows that a single application of a 5% ointment formulation of ketoprofen is effective within 30 minutes to significantly decrease or eliminate all migraine pain. In the case of patient 102, pain rebounded by hour 6. Reapplication of 5% ketoprofen ointment at hour 6 reduced the pain to a level of 2 by 12 hours and at 24 hours the patient was pain free.

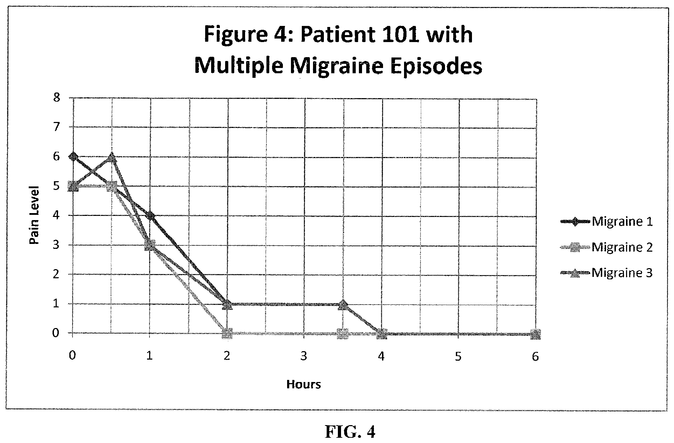

Figure 4 below illustrates results of treating multiple migraine episodes in patient 101. As is shown in this figure, patient 101 was successfully treated for 3 separate migraines and remained pain free for minimally 24 hours after each migraine.

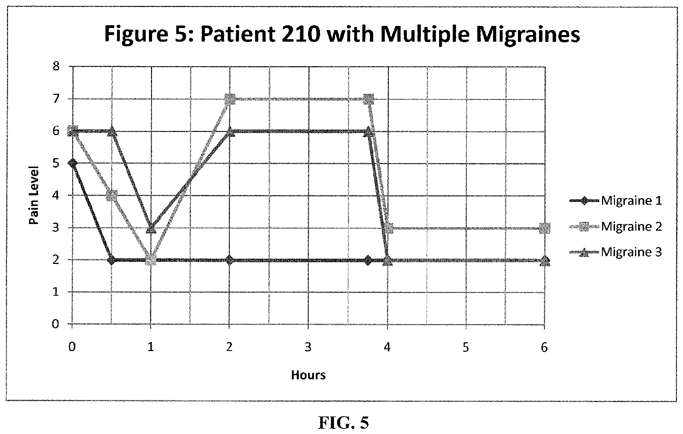

Figure 5 illustrates the results of treating multiple migraine episodes in patient 210. Patient 210 was treated successfully 1 out of 3 times with a single dose treatment of 5% ketoprofen ointment. In two of the three migraines episodes experienced by patient 210, the migraine pain rebounded as shown in the figure and in these two cases the patient reapplied 5% ketoprofen ointment prior to the 4 hour assessment. Upon reapplication, the pain was significantly reduced. This figure teaches that in some cases ketoprofen can and should be reapplied to maintain the patients in a pain-free state.

Although the invention has been described in connection with specific desired embodiments, it should be understood that the invention as claimed should not be unduly limited to such specific embodiments.

## Claims

1. A composition comprising from 1% to 30% (w/w) of ketoprofen and a dermatologically acceptable carrier for use in a method of treating or preventing pain associated with a migraine headache, the method comprising topically administering to a mammal having pain associated with a migraine headache an effective amount of the composition to the sternocleidomastoid muscle at the temporomandibular joint; wherein said composition is formulated as a cream, a gel, an ointment, or a liquid.

2. The composition for use according to claim 1, wherein the composition comprises from 2.5% to 10% (w/w) ketoprofen and a dermatologically acceptable carrier.

3. The composition for use according to claim 1, further comprising from 5% to 20% (w/w) gabapentin.

4. The composition for use according to claim 3, wherein said composition comprises from 2% to 12% (w/w) ketoprofen and from 8% to 12% (w/w) gabapentin.

5. The composition for use according to any one of claims 1-4, wherein said composition further comprises a skin penetrating enhancer.

6. The composition for use according to claim 5, wherein said skin penetrating enhancer is selected from the group consisting of isopropyl myristate, isopropyl palmitate, dimethyl sulfoxide, decyl methyl sulfoxide, dimethylalanine amide of a medium chain fatty acid, dodecyl 2-(N,N-dimethylamino) propionate or salts thereof, tetradecyl(N,N-dimethylamino) acetate, dodecyl(N,N-dimethylamino) acetate, decyl(N,N-dimethylamino) acetate, octyl(N,N-dimethylamino) acetate, and dodecyl(N,N-diethylamino) acetate.

## Patentansprüche

1. Zusammensetzung, umfassend von 1 % bis 30 % (Gew./Gew.) Ketoprofen und einen dermatologisch verträglichen Träger zur Verwendung in einem Verfahren zum Behandeln oder Vorbeugen von Schmerz, der mit einem Migräne-Kopfschmerz assoziiert ist, wobei das Verfahren das topische Verabreichen an einen Säuger, der Schmerz hat, der mit einem Migräne-Kopfschmerz assoziiert ist, einer effektiven Menge der Zusammensetzung an den Sternocleidomastoideus-Muskel am Kiefergelenk ("sternocleidomastoid muscle at the temporomandibular joint") umfasst; wobei diese Zusammensetzung als eine Creme, ein Gel, eine Salbe oder eine Flüssigkeit formuliert ist.

2. Zusammensetzung zur Verwendung gemäß Anspruch 1, wobei die Zusammensetzung von 2,5 % bis 10 % (Gew./Gew.) Ketoprofen und einen dermatologisch verträglichen Träger umfasst.

3. Zusammensetzung zur Verwendung gemäß Anspruch 1, weiterhin umfassend von 5 % bis 20 % (Gew./Gew) Gabapentin.

4. Zusammensetzung zur Verwendung gemäß Anspruch 3, wobei die Zusammensetzung von 2 % bis 12 % (Gew./Gew) Ketoprofen und 8 % bis 12 % (Gew./Gew.) Gabapentin umfasst.

5. Zusammensetzung zur Verwendung gemäß einem beliebigen der Ansprüche 1-4, wobei diese Zusammensetzung weiterhin einen Hautpenetrationverstärker umfasst.

6. Zusammensetzung zur Verwendung gemäß Anspruch 5, wobei der Hautpenetrationsverstärker ausgewählt ist, aus der Gruppe, bestehend aus Isopropylmyristat, Isopropylpalmitat, Dimethylsulfoxid, Decylmethylsulfoxid, Dimethylalaninamid von einer mittelkettigen Fettsäure, Dodecyl-2-(N,N-dimethylamino)-propionat oder Salze davon, Tetradecyl(N,N-dimethylamino)-acetat, Dodecyl(N,N-dimethylamino)-acetat, Decyl(N,N-dimethylamino)-acetat, Octyl(N,N-dimethylamino)-acetat und Dodecyl(N,N-diethylamino)-acetat.

## Revendications

1. Composition comprenant entre 1 % et 30 % (en poids) de kétoprofène et un support dermatologiquement acceptable, destinée à être utilisée dans un procédé de traitement ou de prévention de la douleur associée à une migraine, le procédé consistant à administrer par voie topique à un mammifère ayant une douleur associée à une migraine une quantité efficace de la composition sur le muscle sterno-cléido-mastoïdien au niveau de l'articulation temporo-mandibulaire ; dans laquelle ladite composition est sous la forme d'une crème, d'un gel, d'un onguent, ou d'un liquide.

2. Composition destinée à être utilisée selon la revendication 1, dans laquelle ladite composition comprend entre 2,5 % et 10 % (en poids) de kétoprofène et un support dermatologiquement acceptable.

3. Composition destinée à être utilisée selon la revendication 1, comprenant en outre entre 5 % et 20 % (en poids) de gabapentine.

4. Composition destinée à être utilisée selon la revendication 3, dans laquelle ladite composition comprend entre 2 % et 12 % (en poids) de kétoprofène et entre 8 % et 12 % (en poids) de gabapentine.

5. Composition selon l'une quelconque des revendications 1 à 4, dans laquelle ladite composition comprend en outre un renforçateur de pénétration dans la peau.

6. Composition destinée à être utilisée selon la revendication 5, dans laquelle ledit renforçateur de pénétration dans la peau est sélectionné parmi le groupe constitué par le myristate d'isopropyle, le palmitate d'isopropyle, le diméthylsulfoxide, le décylméthylsulfoxide, le diméthylalaninamide d'un acide gras à chaîne moyenne, le dodécyl-2-(N,N-diméthylamino)propionate ou les sels de celui-ci, le tétradécyl(N,N-diméthylamino)acétate, le dodécyl-(N,N-diméthylamino)acétate, le décyl(N,N-diméthylamino)acétate, l'octyl(N,N-diméthylamino)acétate, et le dodécyl(N,N-diéthyl-amino)acétate.
